# EUROPEAN PATENT SPECIFICATION

(11) **EP 1 572 233 B1**
(45) Date of publication and mention of the grant of the patent: **30.03.2011**
(21) Application number: 03789346.8
(22) Date of filing: 18.12.2003
(51) Int. Cl.: A61K 39/12

(54) **Use of HPV16 and HPV18 as vaccine against one or more of oncogenic HPV type 31, 33, 35, 39, 45, 51, 52, 56, 58, 59, 66, 68**
Verwendung von HPV16 und HPV18 VLPs als Vakzine gegen eine oder mehrere onkogene HPV des Typus 31, 33, 35, 39, 45, 51, 52, 56, 58, 59, 66, 68
Utilisation de HPV16 et de HPV18 comme vaccin contre un ou plusieurs type de HPV oncogène 31, 33, 35, 39, 45, 51, 52, 56, 58, 59, 66, 68

(30) Priority: 20.12.2002 US 435035 P; 20.08.2003 US 496653 P
(43) Date of publication of application: 14.09.2005
(73) Proprietor: GlaxoSmithKline Biologicals s.a., 1330 Rixensart (BE)
(72) Inventor: DUBIN, Gary GlaxoSmithKline, King of Prussia,PA 19 406-2772 (US); INNIS, Bruce GlaxoSmithKline, King of Prussia, PA 19406-2772 (US); SLAOUI, Moncef, M. GlaxoSmithKline, King of Prussia, Pennsylvania 19406 (US); WETTENDORFF, M.A.C. GlaxoSmithKlineBiologicals SA, B-1330 Rixensart (BE)
(74) Representative: Dalton, Marcus Jonathan William
(86) International application number: PCT/EP2003/014562
(87) International publication number: WO 2004/056389

(56) References cited:
- WO-A-01/17551
- WO-A-01/97840
- DE-A- 4 332 596
- US-B1- 6 251 678
- BALSLEY JF ET AL: "Progress in the development of human papillomavirus vaccines for HPV-11 and HPV-16/18 and mapping of a critical neutralizing epitope." 18TH INTERNATIONAL PAPILLOMAVIRUS CONFERENCE 2000, [Online] 2000, XP002278802 Retrieved from the Internet: <URL:http://www.hpv2000.com/idabstract.asp ?id=366> [retrieved on 2004-05-03]
- HPV Clinical Workshop & 20th International Papillomavirus Conference 2002, October 4-9, Paris, Institut Pasteur. Villa-L et al., A dose-ranging safety and immunogenicity study of a quadrivalent HPV (type 6/11/16/18) L1 VLP vaccine in women. XP002281268
- BASS E: "Progress in the Search of a vaccine against human papilloma virus" IAVI REPORT OCTOBER/NOVEMBER 2002, [Online] 10 October 2002 (2002-10-10), XP002281262 Retrieved from the Internet: <URL:www.aegis.com/pubs/iavi/2002/IAVI2002 -1003.html> [retrieved on 2004-04-21]
- Brown-DR et al., A dose-ranging study of the safety and immunogenicity profiles of a quadrivalent HPV (types 6, 11, 16, and 18) L1 VLP candidate vaccine in younghealthy women. ABstr O-51 19th International Papillomavirus Conference, Sept-2001, Florianopolis, Brazil XP002278803
- COMBITA ALBA-LUCIA ET AL: "Identification of two cross-neutralizing linear epitopes within the L1 major capsid protein of human papillomaviruses." JOURNAL OF VIROLOGY. UNITED STATES JUL 2002, vol. 76, no. 13, July 2002 (2002-07), pages 6480-6486, XP002278799 ISSN: 0022-538X
- SCHILLER J T ET AL: "PAPILLOMAVIRUS-LIKE PARTICLE BASED VACCINES: CERVICAL CANCER AND BEYOND" EXPERT OPINION ON BIOLOGICAL THERAPY, ASHLEY, LONDON, GB, vol. 1, no. 4, July 2001 (2001-07), pages 571-581, XP008028179 ISSN: 1471-2598
- STELLER MICHAEL A: "Cervical cancer vaccines: progress and prospects." JOURNAL OF THE SOCIETY FOR GYNECOLOGIC INVESTIGATION. UNITED STATES 2002 SEP-OCT, vol. 9, no. 5, September 2002 (2002-09), pages 254-264, XP002278800 ISSN: 1071-5576
- BACHTIARY BARBARA ET AL: "Impact of multiple HPV infection on response to treatment and survival in patients receiving radical radiotherapy for cervical cancer." INTERNATIONAL JOURNAL OF CANCER. JOURNAL INTERNATIONAL DU CANCER. UNITED STATES 20 NOV 2002, vol. 102, no. 3, 20 November 2002 (2002-11-20), pages 237-243, XP002278801 ISSN: 0020-7136

## Description

The present invention relates to virus-like particles (VLPs) from human papilloma virus (HPV) and to uses thereof in medicine, in particular to providing protection from infection and/or disease caused by heterologous HPV types.

### Background of the Invention

Papillomaviruses are small DNA tumour viruses, which are highly species specific. So far, over 100 individual human papillomavirus (HFV) genotypes have been described. HPVs are generally specific either for the skin (e.g. HPV-1 and -2) or mucosal surfaces (e.g. HPV-6 and -11) and usually cause benign tumours (warts) that persist for several months or years. Such benign tumours may be distressing for the individuals concerned but tend not to be life threatening, with a few exceptions.

Some HPVs are also associated with cancers, known as oncogenic HPV types. The strongest positive association between an HPV and human cancer is that which exists between HPV-16 and HPV-18 and cervical carcinoma. Cervical cancer is the most common malignancy in developing countries, with about 500,000 new cases occurring in the world each year.

Other HPVs of particular interest with respect to cancer are types 31, 33, 35, 39, 45, 51, 52, 56, 58, 59, 66 and 68.

HPV virus like particles (VLPs) have been suggested as potential vaccines for treatment of HPV. Animal studies have shown that VLPs produce no cross protection against infection for other HPV types - see, for example Suzich, J. A, et al, Proc Natl Acad Sci, 92: 11553-11557, 1995, and Breitburd, Seminars in Cancer Biology, vol 9, 1999, pp 431-445.

WO91/17551 discloses that HPV 16 and 18 VLPs can be formulated with 3D-MPL and aluminium hydroxide for vaccination.

There is still a need for a vaccine that protects against multiple HPV types.

The present invention addresses this need.

### Summary of the invention

The invention relates to use of a mixture comprising HPV 16 and HPV 18 VLPs as defined in the claims in the preparation of a medicament for the prevention of infection or disease caused by one or more of the group of oncogenic HPV types, 31,33,35,39,45,51,52,56,58,59,66,68. medicament for the prevention of infection or disease caused by one or more of the

### Detailed Description

We have surprisingly found that use ofHPV 16 and HPV 18 VLPs provides cross protection against infection by one or more of the group of oncogenic HPV types (excluding types 16 and 18), oncogenic types being those types capable of causing cancer. The group of oncogenic types comprises or consists of types 31, 33, 35, 39, 45, 51, 52, 56, 58, 59, 68, 73, 82, 26, 53, and 66.
In particular use of HPV 16 and HPV 18 VLPs provides cross protection against infection and /or disease caused by the group of HPV types 31, 33, 35, 39, 45, 51, 52, 56, 58, 59, 66, and 68, hereinafter called the group of 'high risk cancer types'. Protection against the whole group of high risk cancer types, and against certain specific types and combinations of types has been identified. The effect is in addition to the 'homologous' protective effect seen against HPV 16 and HPV 18 by use of HPV 16 and HPV 18 VLPs respectively. As such, HPV 16 VLPs and HPV 18 VLPs can be used both in a vaccine against HPV 16, HPV 18 and against other HPV types.

Cross protection is herein taken to mean that incidence of infection for a group of oncogenic HPV types (infection being incident or persistent infection) and/or oncogenic disease caused by HPV infection is lower in a group of individuals vaccinated with HPV 16 and/or 18 VLPs, preferably types 16 and 18, than in a non vaccinated group. Complete cross protection against a type, or group of types, is not required in the present invention - indeed, any level of cross protection provides a benefit. Preferably the level of cross protection observed is such that the vaccinated group has 5% less infection and/or disease than a comparable non vaccinated group, more preferably up to 10%, up to 15%, up to 20%, up to 25%, up to 30%, up to 35%, up to 40%, up to 45%, up to 50%, up to 55%, up to 60%, up to 65% up to 70%, up to 80%, up to 90% or even up to 100% less infection and/or disease.

Cross protection may be assessed by detecting the presence of nucleic acid specific for various oncogenic types in the vaccinees and control group. Detection may be carried out, for example, using techniques as described in WO03014402, and references therein, particularly for non-specific amplification of HPV DNA and subsequent detection of DNA types using a LiPA system as described in WO 99/14377, and in Kleter et al, (Journal of Clinical Microbiology (1999), 37 (8): 2508-2517]. Any suitable method can, however, be used for the detection of HPV DNA in a sample, such as type specific PCR using primers specific for each HPV type of interest. Suitable primers are known to the skilled person, or can be easily constructed given that the sequences of the oncogenic HPV types are known.

Suitably cross protection is observed in the female population, preferably women who are seronegative for HPV infection, or seronegative for HPV 16 and 18, preferably adolescent women pre-sexual activity.

Cross protection (as assessed by protection seen in a vaccinated group vs a control group) is preferably seen against any one oncogenic type other than 16 or 18, for example any one of the group of high risk cancer types 31, 33, 35, 39, 45, 51, 52, 56, 58, 59, 66 or 68 or, collectively, groups of high risk cancer types such as any 2,3,4,5,6,7,8, 9,10 or 11 types, or indeed all of the high risk cancer types. All possible combinations of 2,3,4,5,6,7,8,9,10 and 11 of the high risk cancers types are specifically contemplated, and as such there are various different 'groups' of VLP types individualised herein, for which the level of cross protection can be analysed in comparison to a placebo group. Use of HPV 16 and/or HPV 18 VLPS to provide prevention of HPV infection by any such group of HPV types is preferred.

Suitably the present invention provides cross- protection against infection and/or disease by the following preferred groups of HPV types:
- A: the group comprising one or more of HPV types 31, 33, 35, 39, 45, 51, 52, 56, 58, 59, 66,68.
- B: the group of statement A comprising type 31 and one or more of types 33, 35, 39, 45, 51, 52, 56, 58, 59, 66, 68
- C: the group of statement A or B comprising type 33 and one or more of types 35, 39, 45, 51, 52, 56, 58, 59, 66,68
- D: the group of statement A - C comprising type 35 and one or more of types 39, 45, 51, 52, 56, 58, 59, 66,68
- E: the group of statement A-D comprising type 39 and one or more of types 45, 51, 52, 56, 58, 59, 66,68
- F: the group of statement A- E comprising type 45 and one or more of types 51, 52, 56, 58, 59, 66,68
- G: the group of statement A-F comprising type 51 and one or more of types 52, 56, 58, 59, 66,68
- H: the group of statement A-G comprising type 52 and one or more of types 56, 58, 59, 66,68
- I: the group of statement A-H comprising type 56 and one or more of types 58, 59, 66,68
- J: the group of statement A-I comprising type 58 and one or more of types 59, 66,68
- K: the group of statement A-J comprising type 59 and one or more of types 66, 68
- L: the group of statement A-K comprising type 66 and one or more of types 68

In particular we have determined that, across a population, a placebo group was 38% more likely to be infected with at least one of the group of high risk cancer types in comparison to those women vaccinated with a vaccine comprising HPV 16 and 18 VLPs. Thus the invention relates particularly to use of a composition comprising HPV 16 and 18 VLPs in prevention of infection in a population by one or more of the high risk cancer types. Preferably the composition is up to or at least 20% more effective than a placebo in prevention of infection in a population by the group consisting of high risk cancer types, preferably up to or at least 25 % more effective, most preferably up to or at least 30% more effective, suitably up to or at least 34% more effective in prevention of infection, most preferably up to or at least 38 % more effective in prevention of infection.

Preferably the present invention relates to a composition comprising HPV 16 and HPV 18 VLPs as defined in the claims in the preparation of a medicament for the prevention of infection by one or more of HPV 31, 33, 35, 52 and 58.

We have determined that, across a population, a placebo group was 43% more likely to be infected with at least one of the group HPV types 31, 33, 35, 52 and 58 in comparison to those women vaccinated with HPV 16 and HPV 18 VLPs. Thus the invention relates particularly to use of a composition comprising HPV 16 and HPV 18 VLPs in prevention of infection in a population by one or more of HPV 31, 33, 35, 52 and 58. Preferably the combination is up to or at least 10% more effective than a placebo in prevention of infection in a population by the group consisting of HPV 31, 33 35, 52 or 58, more preferably up to or at least 15 % effective, more preferably up to or at least 20% effective, 25% effective, 30% effective, 37% effective or suitably up to or at least 43% effective in prevention of infection.

Preferably the present invention relates to a composition as defined in the claims comprising HPV 16 and HPV 18 VLPs in the preparation of a medicament for the prevention of infection by one or more ofHPV 31, 35 and 58.

We have determined that, across a population, a placebo group was 58% more likely to be infected with at least one of the group of HPV types 31, 35 and 58 in comparison to women vaccinated with HPV 16 and HPV 18 VLPs. Thus the invention relates particularly to use of a composition comprising HPV 16 and HPV 18 VLPs in prevention of infection within a population by one or more of HPV 31, 35 and 58. Preferably the composition is up to or at least 15% more effective than a placebo in prevention of infection in a population by the group consisting of HPV 31, 35 or 58, preferably up to or at least 20 % effective, preferably up to 25% effective, up to 30%, up to 35%, up to 40%, up to 45%, up to 49%, up to 55% or even more effective, suitably up to or at least 58% effective in prevention of infection.

Preferably the present invention relates to a composition as defined in the claims comprising HPV 16 and HPV 18 VLPs in the preparation of a medicament for the prevention of infection by one or more of HPV 45 and 59.

In particular we have determined that, across a population, a placebo group was 33% more likely to be infected with at least one (or more) of the group of HPV types 45 and 59 in comparison to those women vaccinated with a vaccine comprising HPV 16 and 18 VLPs. Thus the invention relates particularly to use of a composition comprising HPV 16 and 18 VLPs in prevention of infection in a population by one or more of HPV 45 and HPV 59. Preferably the composition is up to or at least 20% more effective than a placebo in prevention of infection in a population by the group consisting of HPV 45 and HPV 59, preferably up to or at least 25 % more effective, more preferably up to 30% more effective, more preferably up to or at least 33% more effective in prevention of infection.

Preferably the composition comprising HPV 16 and HPV 18 VLPs provides cross-protection against at least one of HPV 35 and HPV 52 infection.

Preferably the composition of HPV 16 and HPV 18 VLPs also provides protection against infection by HPV 16 and/or HPV 18, preferably both HPV 16 and HPV 18.

Preferably the HPV 16 VLPs in combination with HPV 18 VLPs are used to provide protection against individual oncogenic types, preferably the high risk cancer types, specifically HPV types 31,33,35,39,45,51,52,56,58,59,66,68.

In addition to the cross protection effect, preferably the immunogenic composition of the invention, is used in the active immunization of adults and adolescent females from the age of 10 years onwards to prevent one or more of: HPV-16 and HPV-18 infection, persistent HPV-16 and HPV-18 infection and HPV-16 and HPV-18 associated cervical neoplasia.

Preferably the immunogenic composition disclosed is used to prevent cervical neoplasia associated with infection by other (non HPV 16,18) oncogenic types.

HPV VLPs and methods for the production of VLPs are well known in the art. VLPs typically are constructed from the L1 and optionally L2 structural proteins of the virus, see for example WO9420137 and WO9405792. Any suitable HPV VLP may be used in the present invention which provides cross protection, such as an L1 or L1 + L2 VLP.

Preferably the VLP is an L1 only VLP.

The VLP may comprise full length L1 protein.

Preferably the L1 protein used to form the VLP is a truncated L1 protein. Preferably the truncation removes a nuclear localisation signal. Preferably the truncation is a C terminal truncation. Preferably the C terminal truncation removes less than 50 amino acids, more preferably less than 40 amino acids. Where the VLP is an HPV 16 VLP then preferably the C terminal truncation removes 34 amino acids from HPV 16 L1. Where the VLP is an HPV 18 VLP then preferably the C terminal truncation removes 35 amino acids from HPV 18 L1.

Truncated L1 proteins are suitably functional L1 protein derivatives. Functional L1 protein derivatives are capable of raising an immune response (if necessary, when suitably adjuvanted), said immune response being capable of recognising a VLP consisting of the full length L1 protein and/or the HPV type from which the L1 protein was derived.

VLPs used in the invention as defined in the claims may also comprise other types of functional protein derivatives, including mutants of the full length or truncated HPV L1 proteins such as deletion, substitution, or insertion mutants. Suitable derivatives also include codon optimised sequences. The L1 protein or derivative may also be a fusion proteins, such as the fusion of the L1 protein with L2 or an early protein. The L1 protein or functional protein derivative is able to form a VLP, and VLP formation can be assessed by standard techniques such as, for example, electron microscopy and dynamic laser light scattering.

VLPs may be made in any suitable cell substrate such as yeast cells or insect cells e.g. baculovirus cells, and techniques for preparation of VLPS are well known in the art, such as WO9913056 and US6245568, and references therein.

VLPS are preferably made by disassembly and reassembly techniques, which can provide for more stable and/or homogeneous papillomavirus VLPs. For example, McCarthy et al, 1998 "Quantitative Disassembly and Reassembly of Human Papillomavirus Type 11 Viruslike Particles in Vitro" J. Virology 72(1):33-41, describes the disassembly and reassembly of recombinant L1 HPV 11 VLPs purified from insect cells in order to obtain a homogeneous preparation of VLP's. WO9913056 and US6245568 also describe disassembly/reassembly processes for making HPV VLPs.

Preferably the HPV VLPS used in the invention are made as described in WO9913056 or US6245568

The VLPs used in the invention may be combined with an adjuvant. The vaccines of the invention may comprise a suitable adjuvant or imunostimulant such as, but not limited to, detoxified lipid A from any source and non-toxic derivatives of lipid A, saponins and other reagents capable of stimulating a The type response.

It has long been known that enterobacterial lipopolysaccharide (LPS) is a potent stimulator of the immune system, although its use in adjuvants has been curtailed by its toxic effects. A non-toxic derivative of LPS, monophosphoryl lipid A (MPL), produced by removal of the core carbohydrate group and the phosphate from the reducing-end glucosamine, has been described by Ribi et al (1986, Immunology and Immunopharmacology of bacterial endotoxins, Plenum Publ. Corp., NY, p407-419) and has the following structure:

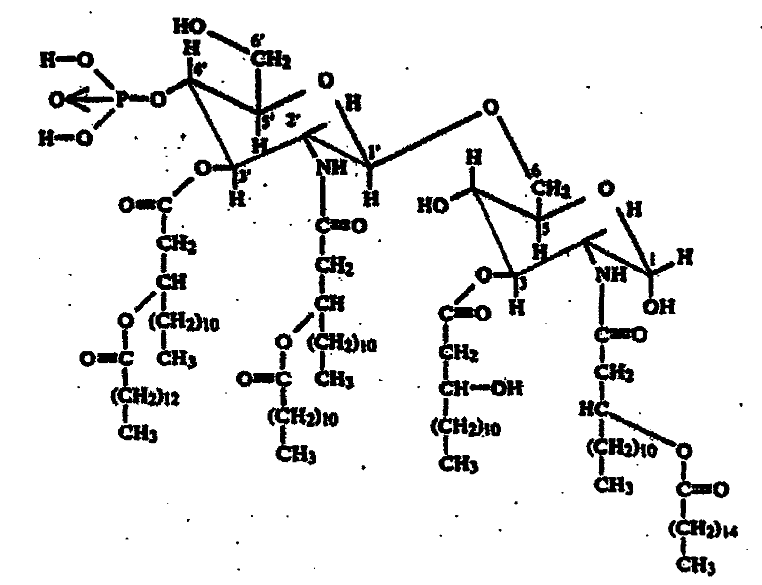

A further detoxified version ofMPL results from the removal of the acyl chain from the 3-position of the disaccharide backbone, and is called 3-O-Deacylated monophosphoryl lipid A (3D-MPL). It can be purified and prepared by the methods taught in GB 2122204B, which reference also discloses the preparation of diphosphoryl lipid A, and 3-O-deacylated variants thereof.

A preferred form of 3D-MPL is in the form of an emulsion having a small particle size less than 0.2µm in diameter, and its method of manufacture is disclosed in WO 94/21292. Aqueous formulations comprising monophosphoryl lipid A and a surfactant have been described in WO9843670A2.

The bacterial lipopolysaccharide derived adjuvants to be formulated in the compositions used in the present invention may be purified and processed from bacterial sources, or alternatively they may be synthetic. For example, purified monophosphoryl lipid A is described in Ribi et al 1986 (supra), and 3-O-Deacylated monophosphoryl or diphosphoryl lipid A derived from *Salmonella sp.* is described in GB 2220211 and US 4912094. Other purified and synthetic lipopolysaccharides have been described (Hilgers et al., 1986, Int.Arch.Allergy.Immunol., 79(4):392-6; Hilgers et al., 1987, Immunology, 60(1):141-6; and EP 0 549 074 B1). A particularly preferred bacterial lipopolysaccharide adjuvant is 3D-MPL.

Accordingly, the LPS derivatives that may be used in the present invention as defined in the claims are those immunostimulants that are similar in structure to that of LPS or MPL or 3D-MPL. In another aspect of the present invention the LPS derivatives may be an acylated monosaccharide, which is a sub-portion to the above structure of MPL.

Saponins are taught in: Lacaille-Dubois, M and Wagner H. (1996. A review of the biological and pharmacological activities of saponins. Phytomedicine vol 2 pp 363-386). Saponins are steroid or triterpene glycosides widely distributed in the plant and marine animal kingdoms. Saponins are noted for forming colloidal solutions in water which foam on shaking, and for precipitating cholesterol. When saponins are near cell membranes they create pore-like structures in the membrane which cause the membrane to burst. Haemolysis of erythrocytes is an example of this phenomenon, which is a properly of certain, but not all, saponins.

Saponins are known as adjuvants in vaccines for systemic administration. The adjuvant and haemolytic activity of individual saponins has been extensively studied in the art (Lacaille-Dubois and Wagner, *supra*)*.* For example, Quil A (derived from the bark of the South American tree Quillaja Saponaria Molina), and fractions thereof, are described in US 5,057,540 and "Saponins as vaccine adjuvants", Kensil, C. R., Crit Rev Ther Drug Carrier Syst, 1996, 12 (1-2):1-55; and EP 0 362 279 B1. Particulate structures, termed Immune Stimulating Complexes (ISCOMS), comprising fractions of Quil A are haemolytic and have been used in the manufacture of vaccines (Morein, B., EP 0 109 942 B1; WO 96/11711; WO 96/33739). The haemolytic saponins QS21 and QS17 (HPLC purified fractions of Quil A) have been described as potent systemic adjuvants, and the method of their production is disclosed in US Patent No.5,057,540 and EP 0 362 279 B1. Other saponins which have been used in systemic vaccination studies include those derived from other plant species such as Gypsophila and Saponaria (Bomford et al., Vaccine, 10(9):572-577, 1992).

An enhanced system involves the combination of a non-toxic lipid A derivative and a saponin derivative particularly the combination of QS21 and 3D-MPL as disclosed in WO 94/00153, or a less reactogenic composition where the QS21 is quenched with cholesterol as disclosed in WO 96/33739.

A particularly potent adjuvant formulation involving QS21 and 3D-MPL in an oil in water emulsion is described in WO 95/17210 and is a preferred formulation.

Accordingly in one embodiment of the present specification there is provided a vaccine adjuvanted with detoxified lipid A or a non-toxic derivative of lipid A, more preferably adjuvanted with a monophosphoryl lipid A or derivative thereof.

Preferably the vaccine additionally comprises a saponin, more preferably QS21.

Preferably the formulation additionally comprises an oil in water emulsion. The present invention also provides a method for producing a vaccine formulation comprising mixing an L2 peptide of the present invention together with a pharmaceutically acceptable excipient, such as 3D-MPL.

Additional components that are preferably present in an adjuvanted vaccine formulation according to the invention include non-ionic detergents such as the octoxynols and polyoxyethylene esters as described herein, particularly t-octylphenoxy polyethoxyethanol (Triton X-100) and polyoxyethylene sorbitan monooleate (Tween 80); and bile salts or cholic acid derivatives as described herein, in particular sodium deoxycholate or taurodeoxycholate. Thus, a particularly preferred formulation comprises 3D-MPL, Triton X-100, Tween 80 and sodium deoxycholate, which may be combined with an L2 antigen preparation to provide a suitable vaccine.

In one preferred embodiment of the present specification, the vaccine comprises a vesicular adjuvant formulation comprising cholesterol, a saponin and an LPS derivative. In this regard the preferred adjuvant formulation comprises a unilamellar vesicle comprising cholesterol, having a lipid bilayer preferably comprising dioleoyl phosphatidyl choline, wherein the saponin and the LPS derivative are associated with, or embedded within, the lipid bilayer. More preferably, these adjuvant formulations comprise QS21 as the saponin, and 3D-MPL as the LPS derivative, wherein the ratio of QS21:cholesterol is from 1:1 to 1:100 weight/weight, and most preferably 1:5 weight/weight. Such adjuvant formulations are described in EP 0 822 831 B.

Suitably the vaccines of the specification are used in combination with aluminium, and are suitably adsorbed or partially adsorbed onto aluminium adjuvants. Suitably the adjuvant is an aluminium salt, preferably in combination with 3D MPL, such as aluminium phosphate and 3D MPL. Aluminium hydroxide, optionally in combination with 3D MPL is also preferred.

Moist preferred in the present invention is the combination of VLPs as defined in the claims with an aluminium salt or with an aluminium salt + 3D MPL. Aluminium hydroxide is preferred as the aluminium salt.

The vaccine may also comprise aluminium or an aluminium compound as a stabiliser.

The vaccines of the specification may be provided by any of a variety of routes such as oral, topical, subcutaneous, mucosal (typically intravaginal), intraveneous, intramuscular, intranasal, sublingual, intradermal and via suppository.

Optionally the vaccine may also be formulated or co-administered with other HPV antigens such as early antigens or non-HPV antigens. Suitably these non-HPV antigens can provide protection against other diseases, most preferably sexually transmitted diseases such as herpes simplex virus, EBV, chlamydia and HIV. We particularly prefer that the vaccine comprises gD or a truncate thereof from HSV. In this way the vaccine provides protection against both HPV and HSV.

The dosage of the vaccine components will vary with the condition, sex, age and weight of the individual, the administration route and HPV of the vaccine. The quantity may also be varied with the number of VLP types. Suitably the delivery is of an amount of vaccine suitable to generate an immunologically protective response. Suitably each vaccine dose comprises 1-100 µg of each VLP, preferably 5-80µg, more preferably 5- 30 µg each VLP, most preferably 5-20 µg of each VLP with 5µg, 6µg, 10µg, 15 µg or 20µg especially preferred.

For all vaccines of the specification, it is preferred that the vaccine is used for the vaccination of adolescent girls aged 10-15, preferably 10-13 years. The vaccine may also be administered to women following an abnormal pap smear or after surgery following removal of a lesion caused by HPV, or who are seronegative and DNA negative for HPV cancer types.

Preferably the vaccine is delivered in a 2 or 3 dose regime, for example in a 0, 1 month regime or 0,1 and 6 month regime respectively. Suitably the vaccination regime incorporates a booster injection after 5 to 10 years, preferably 10 years.

Preferably the vaccine is a liquid vaccine formulation, although the vaccine may be lyophilised and reconstituted prior to administration.

Preferably the immunogen used in the present invention (HPV 16 and HPV 18 VLPs) is used as a vaccine and may be formulated into a vaccine using appropriate excipients. Thus the invention particularly relates to use of an HPV 16 and HPV 18 vaccine, such as an LI only vaccine, in the prevention of HPV µinfection.

The present invention is hereby illustrated by reference to the following non-limiting example.

### Example 1

Healthy women between the ages of 15 and 25 years were immunised with a mixture of HPV 16 and HPV 18 L1 VLPs. The women at enrolment were: 1) seronegative for HPV-16 and HPV-18; 2) negative for high risk HPV infection of the cervix (detected by HPV PCR); 3) had 6 or fewer lifetime sexual partners and 4) had normal PAP smears.

The mixture comprised, per 0.5 ml dose, 20 µg of HPV-16 L1 VLP, 20 µg of HPV-18 L1 VLP and was adjuvanted with 500 µg of aluminum hydroxide and 50 µg of 3D MPL. The placebo group was injected with 500 µg of aluminum hydroxide alone.

The vaccine efficacy (V.E.) against high risk cancer HPV types was assessed, wherein the V.E. is the % improvement in protection against infection by the vaccine compared to a placebo group.

Cross protection was assessed by detecting the presence of nucleic acid specific for various oncogenic types in the vaccinees and control group. Detection was carried out using techniques as described in WO03014402, and references therein, particularly for non-specific amplification of HPV DNA and subsequent detection of DNA types using a LiPA system as described in WO 99/14377, and in Kleter et al, [Journal of Clinical Microbiology (1999), 37 (8): 2508-2517].

Any suitable method can, however, be used for the detection of HPV DNA in a sample, such as type specific PCR using primers specific for each HPV type of interest. Suitable primers are known to the skilled person, or can be easily constructed given that the sequences of the oncogenic HPV types are known.

Vaccine efficacy was assessed against infections for all of the 12 high risk cancer types, HPV-16 phylogenetic-related types (the groups of; 31, 35, and 58; 31, 33, 35, 52 and 58) and HPV-18 phylogenetic related types (45 and 59).

An initial analysis was carried out on an "ITT" (Intention To Treat cohort, representing all individuals who received at least one dose of vaccine). This data is shown in Table 1.

The results presented in Tables 2 and 3 relate to the "ATP" (According To Protocol) group for those patients who complied with all the criteria of the trial. Table 2 is a midpoint analysis with data taken from all patients at the timepoint at which at least 50% of the cohort were 18 months after their first vaccination. Table 3 gives the final results, all data being from subjects at 18 months post first vaccination (month 0). In the ATP group all patients received 3 doses of vaccine at 0,1 and 6 months and were seronegative at 6 months.

As demonstrated by the data presented in table 1, immunization with a mixture of HPV16 and HPV18 VLPs provided apparent cross-protection against other HPV types. At this point the sample sizes are too small to provide for a rigorous statistical analysis, however the data demonstrate a positive trend and suggest that immunization with HPV16 and HPV18 VLPs will be efficacious against infection with other HPV types.

This was confirmed as the study progressed.

Table 2 demonstrates that HPV 16 and HPV 18 provide statistically significant cross protection against the group of high risk cancer types 31,33,35,39,45,51,52,56,58,59, 66 and 68.

Table 3 demonstrates that, except for the HPV-18 related types (which show a very strong trend), there is statistically significant cross-protection against the groups of: HPV 31, 35, 58; HPV 31, 33, 35, 52, 58; and the 12 high risk (non HPV-16/18) types evaluated.

**Table 1**

| HPV types analysed | Number of women infected (vaccine group) | % women infected (vaccine group) **= A** | Number of women infected (placebo group) | % women infected (placebo group) **= B** | % vaccine efficacy 1-(A/B)x 100, adjusted for relative size of vaccine and placebo group | 95% confidence limits -lower limit | 95% confidence limits -upper limit | P |
|---|---|---|---|---|---|---|---|---|
| HPV 31,35, 58 | 5 | 1.1 | 11 | 2.4 | 55.1 | -29.1 | 84.4 | 0.127 |
| HPV 31, 33, 35, 52, 58 | 17 | 3.8 | 24 | 5.4 | 30.3 | -29.7 | 62.6 | 0.252 |
| HPV 45, 59 | 3 | 0.7 | 6 | 1.3 | 50.6 | -97.7 | 87.6 | 0.309 |
| HPV31,33,35, 39,45,51,52,56 ,58,59,66,68. | 27 | 6.3 | 40 | 9.4 | 34.6 | -6.5 | 59.9 | 0.086 |

Samples were taken at 9, 12, 15 and 18 months from patients and tested for HPV infection by the types specified above.

### Table 2 - vaccine efficacy after three doses in preventing incident heterologous infections.

**Table 2: Vaccine efficacy against infection with HPV-16 phylogenetically related types, HPV-18 phylogenetically related types, HPV-16 and/or HPV-18 phylogenetically related types and all high-risk types exclusive of HPV-16 and HPV-18 - ATP cohort (month 6-18)**

| **Infection Type** | **Attack rate** | | | | | | **Vaccine efficacy** | | | |
|---|---|---|---|---|---|---|---|---|---|---|
| | **Vaccine** | | | **Placebo** | | | | | | |
| | **N** | **n** | **AR** | **N** | **n** | **AR** | **%** | **95%** | **CI** | **p-value** |
| HPV-16 related | 433 | 12 | 2.8 | 438 | 24 | 5.5 | 49.4 | 0.2 | 74.4 | 0.060 |
| HPV-16 related* | 423 | 29 | 6.9 | 423 | 46 | 10.9 | 37.0 | 1.6 | 59.6 | 0.052 |
| HPV-18 related | 442 | 9 | 2.0 | 449 | 16 | 3.6 | 42.9 | -27.9 | 74.5 | 0.223 |
| HPV-16/18 related | 433 | 21 | 4.9 | 438 | 41 | 9.4 | 48.2 | 13.8 | 68.9 | 0.012 |
| HPV-16/18 related* | 423 | 34 | 8.0 | 423 | 56 | 13.2 | 39.3 | 9.0 | 59.5 | 0.019 |
| High-risk** | 385 | 53 | 13.8 | 386 | 88 | 22.8 | 39.6 | 17.7 | 55.7 | 0.001 |

| | | | | | | | | | | |
|---|---|---|---|---|---|---|---|---|---|---|
| N = number of subjects in specific cohort n = number of subjects with incident HPV infection AR = Attack rate = n / N 95% CI = 95% confidence interval lower limit = 1- exp (log (arv / arp) + 1.96 * sqrt (1/nv - 1/Nv + 1/np - 1/Np)) upper limit =1- exp (log (arv / arp)-1.96 * sqrt (1/nv-1/Nv + 1/np-1/Np)) when number of cases in vaccine = 0 : lower limit* =1- exp (log (arv* / arp*) + 1.96 * sqrt (1/(nv+0.5)-1(Nv+0.5) + 1/(np+0.5)-1/(Np+0.5))) upper limit* =1- exp (log (arv* / arp*) -1.96 * sqrt (1/(nv+0.5) -1/(Nv+0.5) + µ1/(np+0.5)-1/(Np+0.5))) with: arv = attack rate in vaccine recipients arp = attack rate in placebo recipients nv = number of cases in vaccine recipients Nv = number of cases and non-cases in vaccine recipients np = number of cases in placebo recipients Np = number of cases and non-cases in placebo recipients HPV-16 related: HPV-16 phylogenetically related types 35, 31, 58 without considering other HPV types HPV-16 related*: HPV-16 phylogenetically related types 35, 31, 58, 33, 52 without considering other HPV types HPV-18 related: HPV-18 phylogenetically related types 45, 59 without considering other HPV types HPV-16 and/or HPV-18 related: HPV-16 and/or HPV-18 phylogenetically related types 35, 31, 58, 45, 59 without considering other HPV types HPV-16 and/or HPV-18 related*: HPV-16 and/or HPV-18 phylogenetically related types 35, 31, 58, 33, 52, 45, 59 without considering other HPV types **= High-risk types exclusive of HPV-16 and HPV-18 | | | | | | | | | | |

**Table 3**

| HPV types analysed | Total number of number of subjects with information available per group | Number of women infected (vaccine group) | % women infected (vaccine group) **= A** | Number of women infected (placebo group) | % women infected (placebo group) **= B** | % vaccine efficacy 1- (A/B) x 100, adjusted for relative size of vaccine and placebo group | 95% confidence limits -lower limit | 95% confidence limits -upper limit | P |
|---|---|---|---|---|---|---|---|---|---|
| HPV 31,35, 58 | 412 | 11 | 2.7 | 26 | 6.3 | 57.9 | 15.9 | 78.9 | 0.012 |
| HPV 31, 33, 35, 52, 58 | 403 | 28 | 6.9 | 48 | 12.2 | 43.0 | 11.0 | 63.5 | 0.015 |
| HPV 45, 59 | 421 | 10 | 2.4 | 15 | 3.6 | 33.5 | -46.3 | 69.8 | 0.319 |
| HPV31,33,35, 39,45,51,52,56 ,58,59,66,68. | 368 | 58 | 15.8 | 90 | 25.3 | 37.7 | 16.2 | 53.6 | 0.002 |

Samples were taken at 18 months from patients and tested for HPV infection by the types specified above.

## Claims

1. Use of a composition comprising human papillomavirus (HPV) 16 and HPV 18 virus like particles (VLPs), wherein the VLPs comprise the L1 protein of said HPV or an immunogenic fragment thereof, in the preparation of a medicament for the prevention of infection and/or disease caused by one or more of the group of oncogenic HPV types 31, 33, 35, 39, 45, 51, 52, 56, 58, 59, 66,68.

2. Use according to claim 1 for prevention of infection and/or disease by one or more of the group of HPV 31, HPV 33, HPV 35, HPV 52 and HPV 58.

3. Use according to claim 2 for prevention of infection and/or disease by one or more of the group of HPV 31, HPV 35, and HPV 58.

4. Use according to claim 1 for the prevention of infection and/or disease caused by one or more of the group HPV 45 and 59.

5. Use according to claim 1 for the prevention of infection and/or disease caused by HPV 31.

6. Use according to claim 1 for the prevention of infection and/or disease caused by HPV 45.

7. Use according to claim 1 for the prevention of infection and/or disease caused by HPV 52.

8. Use according to any preceding claim wherein the HPV VLPs are combined with an adjuvant.

9. Use according to claim 8 wherein the adjuvant is a combination of an aluminium salt and 3-O-Deacylated monophosphoryl lipid A (3D-MPL).

10. Use according to claim 9 wherein the adjuvant is a combination of aluminium hydroxide and 3D MPL.

11. Use according to any preceding claim wherein the VLP comprises an L1 protein or immunogenic fragment thereof but no L2 protein.

12. Use according to claim 11 wherein the composition comprises 20 µg HPV 16 L1 VLPs and 20 µg HPV 18 VLPs and the adjuvant comprises 500 µg aluminium hydroxide and 50 µg 3D MPL.

## Patentansprüche

1. Verwendung einer Zusammensetzung, umfassend menschliche Papillomavirus (HPV) 16- und HPV 18-virusartige Partikel (VLPs), wobei die VLPs das L1-Protein dieser HPV oder ein immunogenes Fragment davon umfassen, zur Herstellung eines Medikaments zur Prävention einer Infektion und/oder Erkrankung, die durch einen oder mehrere aus der Gruppe der onkogenen HPV-Typen 31, 33, 35, 39, 45, 51, 52, 56, 58, 59, 66, 68 verursacht wird.

2. Verwendung gemäß Anspruch 1 zur Prävention einer Infektion und/oder Erkrankung durch eines oder mehrere aus der Gruppe HPV 31, HPV 33, HPV 35, HPV 52 und HPV 58.

3. Verwendung gemäß Anspruch 2 zur Prävention einer Infektion und/oder Erkrankung durch eines oder mehrere aus der Gruppe HPV 31, HPV 35 und HPV 58.

4. Verwendung gemäß Anspruch 1 zur Prävention einer Infektion und/oder Erkrankung durch eines oder mehrere aus der Gruppe HPV 45 und HPV 59.

5. Verwendung gemäß irgendeinem Anspruch 1 zur Prävention einer Infektion und/oder Erkrankung verursacht durch HPV 31.

6. Verwendung gemäß Anspruch 1 zur Prävention einer Infektion und/oder Erkrankung verursacht durch HPV 45.

7. Verwendung gemäß Anspruch 1 zur Prävention einer Infektion und/oder Erkrankung verursacht durch HPV 52.

8. Verwendung gemäß irgendeinem vorhergehenden Anspruch, wobei HPV VLPs mit einem Adjuvans kombiniert werden.

9. Verwendung gemäß Anspruch 8, wobei das Adjuvans eine Kombination aus einem Aluminiumsalz und 3-O-deacetyliertem Monophosphoryllipid A (3D-MPL) ist.

10. Verwendung gemäß Anspruch 9, wobei das Adjuvans eine Kombination aus Aluminiumhydroxid und 3-D-MPL ist.

11. Verwendung gemäß irgendeinem vorhergehenden Anspruch, wobei das VLP ein L1-protein oder immunogenes Fragment davon, aber kein L2-Protein umfasst.

12. Verwendung gemäß Anspruch 11, wobei die Zusammensetzung 20 µg HPV 16 L1 VLPs und 20 µg HPV 18 VLPs umfasst und das Adjuvans 500 µg Aluminiumhydroxid und 50 µg 3D-MPL umfasst.

## Revendications

1. Utilisation d'une composition comprenant un papillomavirus humain (HPV) 16 et des particules semblables à un virus HPV 18 (VLP) où les VLP comprennent la protéine L1 dudit HPV ou un fragment immunogène de celle-ci, dans la préparation d'un médicament pour la prévention d'une infection et/ou de maladies provoquées par un ou plusieurs groupes de HPV oncogène de types 31, 33, 35, 39, 45, 51, 52, 56, 58, 59, 66, 68.

2. Utilisation selon la revendication 1 pour la prévention d'une infection et/ou d'une maladie par un ou plusieurs des groupes HPV 31, HPV 33, HPV 35, HPV 52 et HPV 58.

3. Utilisation selon la revendication 2, pour la prévention d'une infection et/ou d'une maladie par un ou plusieurs des groupes HPV 31, HPV 35 et HPV 58.

4. Utilisation selon la revendication 1, pour la prévention d'une infection et/ou d'une maladie causée par un ou plusieurs des groupes HPV 45 et 59.

5. Utilisation selon la revendication 1 pour la prévention d'une infection et/ou d'une maladie provoquée par le HPV 31.

6. Utilisation selon la revendication 1, pour la prévention de l'infection et/ou d'une maladie provoquée par le HPV 45.

7. Utilisation selon la revendication 1, pour la prévention d'une infection et/ou d'une maladie provoquée par le HPV 52.

8. Utilisation selon l'une quelconque des revendications précédentes, dans laquelle les VLP HPV sont combinées à un adjuvant.

9. Utilisation selon la revendication 8, dans laquelle l'adjuvant est une combinaison d'un sel d'aluminium et d'un monophosphoryl-lipide A 3-O-désacylé (3D-MPL).

10. Utilisation selon la revendication 9, dans laquelle l'adjuvant est une combinaison d'hydroxyde d'aluminium et d'un 3D MPL.

11. Utilisation selon l'une quelconque des revendications précédentes, dans laquelle les VLP comprennent une protéine L1 ou un fragment immunogène de celle-ci mais aucune protéine L2.

12. Utilisation selon la revendication 11, dans laquelle la composition comprend 20 µg de VLP L1 de HPV 16 et 20 µg de VLP de HPV 18 et l'adjuvant comprend 500 µg d'hydroxyde d'aluminium et 50 µg de 3D MPL.
